# EUROPÄISCHE PATENTSCHRIFT

(11) **EP 2 182 772 B1**
(45) Veröffentlichungstag und Bekanntmachung des Hinweises auf die Patenterteilung: **03.06.2015**
(21) Anmeldenummer: 09173933.4
(22) Anmeldetag: 23.10.2009
(51) Int. Cl.: H05B 3/34, A41D 13/005, A41D 15/00

(54) **Elektrisch heizbares Kleidungsstück**
Electrically heatable item of clothing
Pièce de vêtement pouvant être chauffée électriquement

(30) Priorität: 03.11.2008 DE 102008043426
(43) Veröffentlichungstag der Anmeldung: 05.05.2010
(73) Patentinhaber: BSH Hausgeräte GmbH, 81739 München (DE)
(72) Erfinder: Fux, Constanze, 80689 München (DE); Hafer, Christian, Dr., 85435 Erding (DE); Klemp, Eric, Dr., 86899 Landsberg am Lech (DE)

(56) Entgegenhaltungen:
- WO-A1-83/02562
- US-A- 3 988 568
- US-A- 4 512 830
- US-A- 5 302 806
- US-A1- 2008 093 354
- US-B1- 6 329 638

## Beschreibung

Die Erfindung betrifft ein elektrisch heizbares Kleidungsstück mit Heizelementen, wie es beispielsweise aus der US 1,358,509 als bekannt hervorgeht.

Elektrisch heizbare Kleidungsstücke sind bekannt. Sie enthalten typischerweise einen elektrisch leitfähigen Draht, in dem aufgrund seines ohmschen Widerstands Joulesche Wärme erzeugt wird, wenn er von einem elektrischen Strom durchflossen wird. Der Draht ist mit einem Trägergewebe des Kleidungsstücks verbunden; je nachdem, wie der Draht im oder am Trägergewebe geführt sind, können ausgewählte Bereiche des Kleidungsstücks selektiv oder flächig beheizt werden.

Die US 1,358,509 beschreibt eine solche elektrisch beheizbare Bekleidung mit mehreren Einzelstücken (Jacke, Hose, Ärmel, ...). Jedes dieser Bekleidungsstücke besteht aus einem Trägergewebe mit eingewebten oder aufgenähten Heizdrähten bzw. Heizwendeln, die über Kabel mit den Heizdrähten bzw. Heizwendeln der anderen Bekleidungsstücke verbunden werden können, um wahlweise den ganzen Körper oder ausgewählte Körperteile zu wärmen. In jedem einzelnen Bekleidungsstück ist der Verlauf der Heizdrähte im Trägermaterial auf das spezifische Kleidungsstück zugeschnitten, was die Herstellung solcher Bekleidung sehr aufwendig macht und zur Folge hat, dass diese Bekleidungsstücke entweder Unikate (d.h. auf die Körperform eines spezifischen Benutzers zugeschnitten) sind oder in einer Standardgröße vorliegen (d.h. für die allermeisten Benutzers entweder zu weit oder zu eng sind). Die Bekleidung dient als heizbare Oberbekleidung bzw. Überzug, insbesondere für Flieger. Eine ähnliche heizbare Ganzkörperbekleidung ist auch aus der US 3,999,037 bekannt.

Weiterhin ist in der US 2,329,766 eine mehrstückige beheizbare Fliegerbekleidung gezeigt, deren Einzelstücke aus einem mehrlagigen Trägergewebe bestehen. Zur Beheizung ist an dem Trägergewebe streifenförmiges Heizband aufgenäht, in das Widerstandsdrähte eingewoben sind. Die Verwendung standardisierten Heizbandes gestattet es, mit geringem Aufwand Fliegerbekleidung in verschiedenen Konfektionsgrößen herzustellen. Allerdings handelt es sich auch in diesem Fall um Kleidungsstücke mit einer universellen Passform, die nicht geeignet sind, sich der individuellen Körperform ihres Benutzers anzupassen. Weiterhin sind die in den genannten Schriften gezeigten beheizbaren Kleidungsstücke aufgrund ihres technischen Aufbaus vergleichsweise unflexibel.

Die US 2008/0093354 offenbart eine elektrothermische Weste mit einer Außenschicht, einer Innenschicht und einem elektrothermischen Element. Die US 6,329,638 offenbart eine Wärmeweste mit einer Wärme- und einer Vibrationseinrichtung. Die US 3,988,568 offenbart eine beheizbare Kopfbedeckung. Die US 5,302,806 offenbart eine Wärmeweste mit einer auf einer chemischen Reaktion basierenden Heizeinrichtung. US 4,512,830 offenbart eine elektrisch betreibbare Heizkappe; und die WO 83/02562 offenbart schließlich ein auf Basis eines Wärmetauschers funktionierendes Temperatursteuersystem für den menschlichen Körper.

Der Erfindung liegt die Aufgabe zugrunde, ein bekanntes elektrisch beheizbares Kleidungsstück in einer solchen Weise weiterzuentwickeln, dass eine schnelle und einfache Anpassung des Kleidungsstücks an unterschiedliche Konfektionsgrößen und Körpermaße ermöglicht wird.

Die Aufgabe wird durch die Merkmale des Anspruchs 1 gelöst. Vorteilhafte Ausgestaltungen sind Gegenstand der Unteransprüche.

Danach umfasst das elektrisch heizbare Kleidungsstück Heizelemente aus einem elektrisch leitfähigen Heizgewebe sowie Fixierelemente, die eine Variation der Form und/oder der Größe des Kleidungsstücks gestatten und Kontaktierelemente zum elektrischen Verbinden benachbarter Heizelementen wobei die Kontaktierelemente des Heizelements mit den diesem Heizelement zugeordneten Fixierelementen kurzgeschlossen sind.

Mit Hilfe dieser Fixierelemente kann das Kleidungsstück in Bezug auf seine Länge und/oder Breite variiert werden, indem z.B. Bereiche des Kleidungsstücks lösbar gerafft bzw. eingefaltet werden und/oder zusätzliche Heizelemente in das Kleidungsstück eingefügt werden. Die zusätzlichen Heizelemente werden mit Hilfe von als Befestigungsmitteln ausgestalteten Fixiermitteln, insbesondere Reißverschlüssen, Knöpfen, Druckknöpfen und/oder Klettmaterialien am Kleidungsstück befestigt: sie können beispielsweise an die Innenwand (Futter) des Kleidungsstücks angeknöpft werden (wodurch bei gleich bleibender Außenkontur die lokale Wanddicke des Kleidungsstücks erhöht und somit der Innenraum reduziert wird) oder mit Hilfe von Reißverschlüssen in einen teilbaren Bereich des Kleidungsstücks eingefügt werden (wodurch sowohl die Außen- als auch die Innenkontur des Kleidungsstücks verändert wird).

Vorteilhafterweise besteht der zu heizende Teil der Heizelemente aus einem elektrisch leitfähigen Heizgewebe; solchermaßen gestaltete Heizelemente sind flexibel und dünnwandig und lassen sich daher leicht in ein Kleidungsstück integrieren.

In einer vorteilhaften Ausgestaltung sind die Fixierelemente durch Reißverschlüsse gebildet, deren Seitenteile beabstandet zueinander auf der Außen- oder Innenseite des Kleidungsstücks aufgenäht sind. Beim Schließen des Reißverschlusses wird der zwischen den Reißverschluss-Seitenteilen liegende Bereich des Kleidungsstücks gerafft und das Kleidungsstück dadurch - je nach Lage und Anordnung des Reißverschlusses enger oder kürzer gemacht. Durch Öffnen bzw. Schließen des Reißverschlusses kann das Kleidungsstück schnell und einfach in seiner Gesamtbreite bzw. Gesamtlänge verändert werden. Alternativ können Reißverschlüsse vorgesehen sein, mit deren Hilfe ein zusätzliches Heizelement in das Kleidungsstück eingefügt werden kann, um die Breite bzw. Länge des Kleidungsstücks zu vergrößern.

Erfindungsgemäß umfasst das Kleidungsstück mehrere Heizelemente, die elektrisch miteinander verbunden werden können. Hierzu sind auf den einzelnen Heizelementen Kontaktierelemente vorgesehen, mit deren Hilfe die Heizelemente mit benachbarten Heizelementen (parallel oder in Serie) verbunden werden können. Dies ermöglicht es, bei einer Größenreduktion des Kleidungsstücks einzelne Heizelemente aus dem Kleidungsstück herauszunehmen bzw. Heizbereiche, die in einem gerafften Bereich des Kleidungsstücks angeordnet sind, gezielt auszuschalten, um eine lokale Überheizung des Kleidungsstücks zu vermeiden. Die Heizelemente können sich in Bezug auf ihre spezifische Heizleistung unterscheiden, so dass in Körperbereichen, die stärker geheizt werden sollen, Heizungselemente mit einer höheren Heizleistung vorgesehen sind als in anderen Körperbereichen. Die Heizungselemente enthalten typischerweise eine Anordnung von Drähten bzw. Metallfäden die in Reihe oder in Serie geschaltet sind und über Kabel mit einer Stromversorgungeinheit verbunden werden können. Eine Variation der spezifischen Heizleistung der Heizelemente kann daher durch eine Variation der Dichte oder der spezifischen Widerstände der Drähte in den Heizelementen erreicht werden.

Die Fixierelemente, mit deren Hilfe die Größe und/oder die Form des Kleidungsstücks variiert werden kann, bestehen zumindest abschnittsweise aus einem elektrisch leitfähigen Werkstoff. Die Heizungselemente, die in den zu variierenden Bereichen des Kleidungsstücks vorgesehen sind, weisen Kontaktelemente auf, mit denen diese Heizelemente elektrisch an eine elektrische Stromversorgung bzw. an benachbarte Heizelemente angekoppelt werden können; vorteilhafterweise sind diese Kontaktelemente mit den Fixierelementen verbunden bzw. bilden Teil der Fixierelemente. Auf diese Weise werden bei einer Größen- bzw. Formveränderung des Kleidungsstücks die betreffenden Heizungselemente automatisch elektrisch angeschlossen bzw. ausgeschaltet.

Wenn an dem Kleidungsstück Befestigungsmittel zur lösbaren Befestigung zusätzlicher Heizelemente vorgesehen sind, so ist es vorteilhaft, diese Befestigungsmittel elektrisch mit den Kontaktelementen der Heizelemente zu verbinden, um die zusätzlichen Heizelemente an weitere im Kleidungsstück vorgesehene Heizelemente anzuschließen und auf diese Weise automatisch in den Stromkreislauf der Kleidungsheizung einzubinden.

Im folgenden wird die Erfindung anhand mehrerer in den Figuren dargestellter Ausführungsbeispiele näher erläutert. Dabei zeigen:

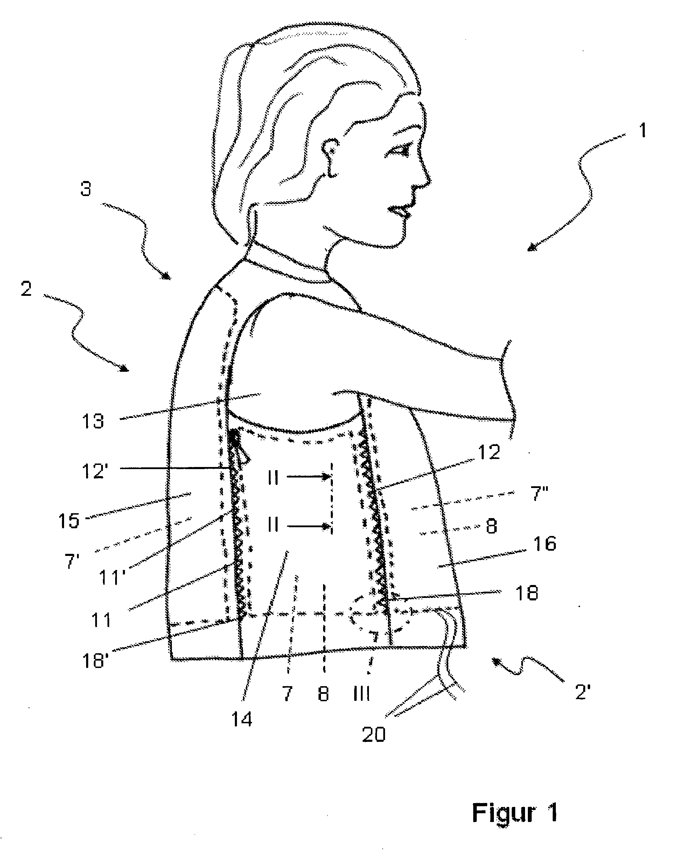

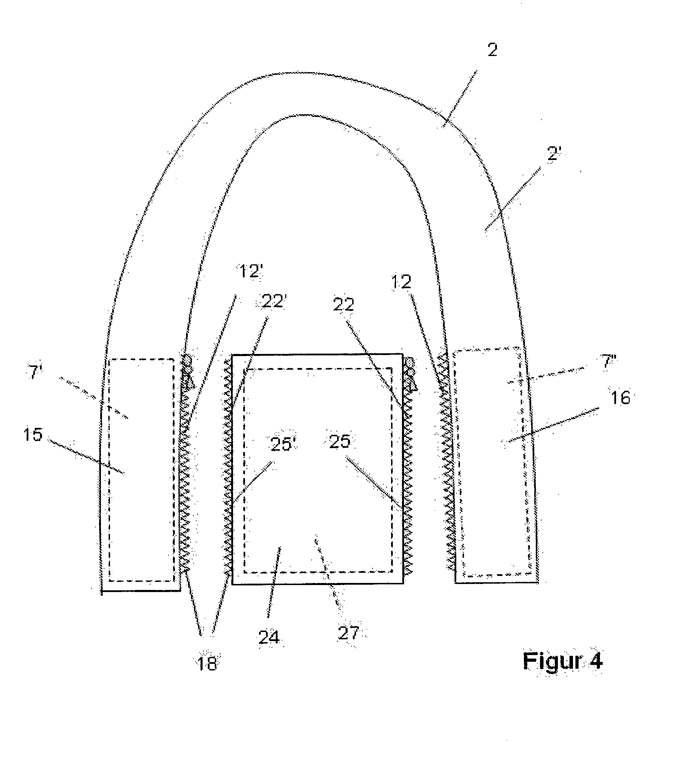

- Fig.4: eine Explosionsdarstellung des Kleidungsstücks der Figur 1 mit einem einfügbaren Zusatz-Heizelement.

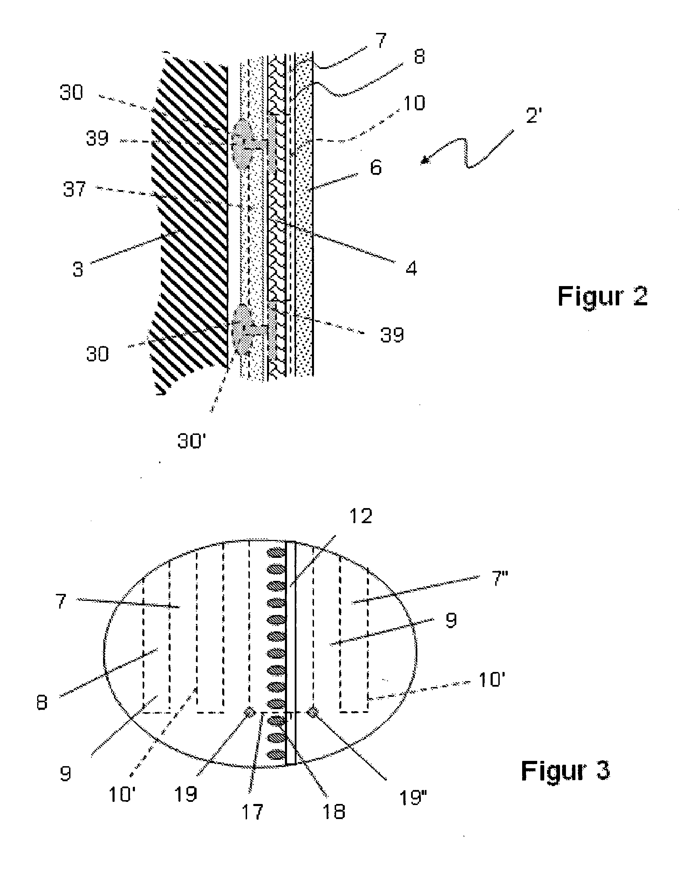

Figur 1 zeigt eine schematische Seitenansicht einer Person 1, die mit einer elektrisch beheizbaren Weste 2' (als Beispiel eines elektrisch heizbaren Kleidungsstücks 2) bekleidet ist. Wie aus der Schnittansicht der Figur 2 hervorgeht, umfasst die Weste 2' eine dem Körper 3 zugewandte Innenschicht 4 und eine körperferne Außenschicht 6, zwischen denen Heizelemente 7, 7', 7" aus einem elektrisch leitfähigen Heizgewebe 8 angeordnet ist. Die Heizelemente 7, 7', 7" sind mit der Innen- und/oder der Außenschicht 4, 6 verbunden (beispielsweise durch eine Naht). Die Innenschicht 4 und die Außenschicht 6 können aus einem elastischen oder inelastischen Textil aus Natur- oder Kunstfaser bestehen, das beispielsweise durch Weben, Stricken oder Wirken hergestellt ist. Weiterhin können die Innen- und Außenschicht 4, 6 aus einer elastischen oder inelastischen Folie oder aus einem Vlies bestehen. Die körperferne Außenschicht 6 kann wind- und/oder wasserabweisende Eigenschaften aufweisen.

Wie in der Detaildarstellung der Figur 3 schematisch gezeigt ist, besteht das Heizgewebe 8 der Heizelemente 7, 7', 7" aus einem flächenhaften Textil oder einer Folie, in die elektrisch leitende Drähte oder Filamente eingewebt oder in anderer Weise integriert sind. Im vorliegenden Ausführungsbeispiel ist das Heizgewebe 8 eine Polyesterfolie 9, in die Heizdrähte 10 in Form beschichteter Metallfäden 10' eingebettet sind. Die Metallfäden 10' sind mit einer elektrisch isolierenden Beschichtung versehen, die elektrische Kurzschlüsse zwischen den Metallfäden 10' verhindert. Solche Heizgewebe 8 mit sehr geringem spezifischen Gewicht (< 100 g / m²) sind bekannt und kommerziell erhältlich (z.B. SEFAR Power Heat). Zur elektrischen Kopplung der Metallfäden 10' an eine (in den Figuren nicht dargestellte) Stromversorgungseinheit sind die Metallfäden 10' in einem Anfangs- und einem Endbereich 13, 14 über eine Verbindungseinrichtung 15 (beispielsweise in Form einer Metallfolie) elektrisch miteinander verschaltet. Mehrere individuelle Metallfäden 10' können zu Heizbändern zusammengefasst sein, was die Verschaltung der Metallfäden 10' erleichtert. Die Flächendichte und der spezifische Widerstand der Metallfäden 10' kann für die verschiedenen Heizelemente 7, 7', 7" unterschiedlich sein, so dass die Heizelemente 7, 7', 7" unterschiedliche spezifische Heizleistungen (in W/cm²) haben können. Weiterhin kann die Flächendichte und der spezifische Widerstand der Metallfäden 10' innerhalb des Heizelements 7, 7,' 7" variabel sein, wodurch eine gezielt inhomogene Wärmeverteilung über das Heizelement 7, 7,' 7" hinweg erreicht werden kann. - Alternativ kann das Heizgewebe 8 beispielsweise eine durch Weben oder Wirken hergestellte Textilware sein, in die dünne Heizdrähte 10 eingewoben oder eingewirkt oder anderweitig befestigt sind.

Zur Beheizung der Weste 2' werden die Heizelemente 7, 7', 7" an eine (in den Figuren nicht gezeigte) Stromversorgungseinheit angeschlossen. Hierzu kann jedes Heizelement 7, 7', 7" - z.B. mit Hilfe von Kabeln, die zwischen Innen- und Außenschicht 4, 6 der Weste geführt werden - einzeln mit der Stromversorgungseinheit verbunden werden. Vorteilhafter ist es jedoch, wenn die Heizdrähte 10 jedes Heizelements 7 - wie in Figur 3 gezeigt - parallel oder in Serie mit den benachbarten Heizelementen 7" verschaltet werden: In diesem Fall fällt nur ein einziges Kabelpaar 20 an, das mit der Stromversorgungseinheit verbunden werden muss. Zur Verbindung mit benachbarten Heizelementen 7', 7" sind die Heizelemente mit Kontaktierelementen 19, 19', 19" versehen. Im vorliegenden Ausführungsbeispiel sind die Heizelemente 7, 7', 7" in Serie geschaltet und bestehen aus einem Streifen aus Heizgewebe 8, der die Weste 2' über ihren gesamten Umfang überspannt.

Um den Umfang der Weste 2' variieren zu können (und somit auf Personen 1 unterschiedlicher Körpermaße bzw. Konfektionsgrößen einstellen zu können), sind an der Weste 2' Fixierelemente 11 vorgesehen, mit deren Hilfe der Stoff der Weste 2' lokal gerafft bzw. eingefaltet werden kann. Im Ausführungsbeispiel der Figuren 1 und 3 sind diese Fixierelemente 11 durch Reißverschlüsse 11' gebildet: Auf der Außenschicht 6 der Weste 2' ist in einem Unterachselbereich 14 unterhalb des Armausschnitts 13 ein Reißverschluss 11' angeordnet, dessen Seitenteile 12, 12' beabstandet zueinander angeordnet sind. Im geöffneten Zustand der unter beiden Armausschnitten 13 angeordneten Reißverschlüsse 11' hat die Weste 2' einen Gesamtumfang, der sich aus der Breite des Rückenteils 15, des Brustteils 16 und der beiden Unterachselbereiche 14 zusammensetzt. Im geschlossenen Zustand der Reißverschlüsse 11' ist der zwischen den Seitenteilen 12, 12' des Reißverschlusses 11' liegende Unterachselbereich 14 der Weste 2' nach innen eingefaltet, so dass der Gesamtumfang der Weste 2' sich nur aus der Breite des Rückenteils 15 und des Brustteils 16 zusammensetzt. Durch Öffnen und Schließen der Reißverschlüsse 11' kann die Weste 2' also auf einen geringeren oder größeren Umfang eingestellt werden. Werden keine Abhilfsmaßnahmen getroffen, so wird im eingefalteten Zustand des Unterachselbereichs 14 dieser Bereich besonders stark beheizt, da dort mehrere Schichten des Heizgewebes 8 vorliegen. Um eine solche inhomogene (d.h. verstärkte) Beheizung im Unterachselbereich 14 zu unterbinden, wird das dem Unterachselbereich 14 zugeordnete Heizelement 7 im eingefalteten Zustand kurzgeschlossen. Dies erfolgt dadurch, dass eine elektrische Verbindung 17, die das Heizelement 7 des Unterachselteils 14 an das Heizelement 7" des Brustteils 16 anschließt, mit mindestens einem metallischen Krampen 18 des Reißverschluss-Seitenteils 12 verbunden ist; analog ist die elektrische Verbindung 17', die das Heizelement 7 des Unterachselteils 14 an das Heizelement 7" des Rückenteils 15 anschließt, mit mindestens einem metallischen Krampen 18' des gegenüberliegenden Reißverschluss-Seitenteils 12' verbunden. Im geöffneten Zustand des Reißverschlusses 11' sind die Reißverschluss-Seitenteile 12, 12' zueinander beabstandet, so dass der Heizstrom das zwischen Brust- und Rücken-Heizelementen 7', 7" geschaltete Unterachsel-Heizelement 7 durchfließt. Ist der Reißverschluss 11' jedoch geschlossen, so wird durch den Kontakt der metallischen Reißverschluss-Seitenteile 12, 12' das Unterachsel-Heizelement 7 kurzgeschlossen, so dass in diesem Bereich keine Beheizung erfolgt.

Soll eine Reduktion des Westenumfangs in mehreren Stufen ermöglicht werden, so können zusätzlich zu dem Reißverschluss-Seitenteil 12 weitere gestuft und parallel dazu angeordnete Reißverschluss-Seitenteile vorgesehen sein, die wahlweise mit dem Seitenteil 12' zu einem geschlossenen Reißverschluss kombiniert werden können.

Um die oben beschriebene Größen- bzw. Maßänderung des Kleidungsstücks 2 zu erreichen, die von einem selektiven elektrischen Kurzschluss von Heizelementen begleitet wird, können anstelle eines metallischen Reißverschlusses beispielsweise auch metallische Druckknöpfe o.ä. verwendet werden. Alternativ bzw. zusätzlich zu der gezeigten Anordnung der Fixierelemente 11 an der Außenseite 6 des Kleidungsstücks 2 können die Fixierelemente 11 auch an der Innenseite 4, beispielsweise als Teil des Futters des Kleidungsstücks 2 vorgesehen werden.

Wurde bei der in Figur 1 gezeigten Weste 2' die Maßanpassung dadurch bewirkt, dass ein fest mit der Weste 2' verbundener Bereich 14 eingefaltet (und das zugehörige Heizelement 7 ggf. elektrisch abgekoppelt) wird, so kann alternativ die Reduktion des Westenumfangs auch dadurch bewirkt werden, dass ein lösbar in der Weste 2' befestigtes Zusatz-Heizelement 27 entfernt wird. Dies ist in Figur 4 in einer Explosionszeichnung gezeigt: Ein einfügbares Unterachselstück 24 mit einem Heizelement 27 ist an seinen einander gegenüberliegenden Rändern 25, 25' mit Reißverschluss-Seitenteilen 22, 22' versehen, die mit Reißverschluss-Seitenteilen 12, 12' auf dem Brust- und Rückenteil 15, 16 kombiniert werden können, um eine Weste 2' mit großem Westenumfang zu bilden. Über ausgewählte Krampen 18 der Reißverschluss-Seitenteile 12, 12', 22, 22', die mit den Heizdrähten 10 der Heizelemente 7', 7", 27 verbunden sind, werden die Heizelemente 7', 7", 27 über die ineinander verschränkten Reißverschluss-Seitenteile 12', 22' bzw. 12, 22 elektrisch in Serie verbunden. Um eine Weste 2' mit kleinerem Westenumfang zu bilden, wird das Unterachselstück 24 durch Öffnen der Reißverschlüsse aus der Weste 2' herausgelöst, wobei dann die Heizelemente 7', 7" von Brust- und Rückenteil 15, 16 über die verschränkten Reißverschluss-Seitenteile 12, 12' elektrisch in Serie geschaltet sind.

Um den Umfang der Weste 2' zu reduzieren, können alternativ zu den Reißverschlüssen bzw. Druckknöpfen können auf der Außenseite 6 der Weste 2' Klettverschlüsse (d.h. Nylonstreifen mit Schlaufen bzw. mit Widerhaken) vorgesehen sein, die es ermöglichen, Bereiche der Weste 2' zumindest einzufalten und in diesem Zustand zu fixieren. Alternativ kann dies auch mit Hilfe von Knöpfen erfolgen. Weiterhin können auf der Innenseite 4 der Weste 2' - wie gestrichelt in Figur 2 angedeutet - Befestigungsmittel 30 zur Befestigung zusätzlicher (lösbar angeordneter) Heizelemente 37 vorgesehen sein. Als Befestigungsmittel 30 können insbesondere Klettstreifen, Druckknöpfe 30' oder Knopfleisten verwendet werden. Wenn die Befestigungsmittel 30 metallische Kontaktierelemente 39, umfassen, die mit ausgewählten Heizdrähten 10 der Heizelemente 7 bzw. 37 verbunden sind, so können die Zusatz-Heizelemente 37 - wie oben beschrieben - beim Einknöpfen automatisch an die Stromversorgung der elektrisch beheizbaren Weste 2' angeschlossen werden.

Wird das heizbare Kleidungsstück 2 von einer großen bzw. breiten Person 1 benutzt, so muss Sorge getragen werden, dass das Heizgewebe 8 der Heizelemente 7, 7', 7" keine zu starke Dehnung erfährt: Wird das Heizgewebe 8 nämlich zu stark gedehnt, so besteht die Gefahr, dass die im Heizgewebe 8 enthaltenen elektrischen Leiter 10 überdehnt werden und/oder reißen, was zu Schädigungen der elektrischen Leitfähigkeit bis hin zu einem vollständigen Funktionsverlust des heizbaren Kleidungsstücks 2 führen kann. Um solche Überdehnungen des Heizgewebes 8 zu vermeiden und eine faltenfreie, angenehme Passform des heizbaren Kleidungsstücks 2 sicherzustellen, sind die Heizelemente 8 vorteilhafterweise in einer solchen Weise mit einem elastischen Trägergewebe 5 verbunden bzw. an einem solchen befestigt, dass starke Dehnungen des Kleidungsstücks 2 durch das elastische Trägergewebe 5 aufgefangen bzw. kompensiert werden. Insbesondere kann das Heizgewebe 8 der Heizelemente 7, 7', 7" in einer solchen Weise auf elastisches Trägergewebe aufgenäht sein, dass im ungedehnten Zustand des Trägergewebes 5 das Heizgewebe 8 gegenüber dem Trägergewebe 5 gefältelt ist. Wird das Kleidungsstück 2 gedehnt (z.B. indem der Benutzer 1 des Kleidungsstücks 2 eine entsprechende Bewegung ausführt oder wenn das Kleidungsstück 2 von einer Person 1 mit größerem Körperumfang getragen wird), dann wird das gefältelte Heizgewebe 8 gestreckt, wobei die Fältelung abnimmt und das Heizgewebe 8 flach auf dem Trägergewebes 5 aufliegt. Die Fältelung ist so bemessen, dass das Heizgewebe 8 beim Erreichen der Dehngrenze des Trägergewebes 5 glatt auf dem Trägergewebe 5 anliegt, aber frei von mechanischen Spannungen ist.

Alternativ bzw. zusätzlich zu den oben beschriebenen Fixierelementen 11 in Form von Reißverschlüssen 11' und Befestigungsmitteln 30 in Form von Klettstreifen, Druckknöpfen 30' oder Knopfleisten können zur Anbindung der Heizelemente 27, 37 beispielsweise Haken/Ösen verwendet werden, oder die Heizelemente 27, 37 können mit Hilfe von Schnüren, die durch Ösen geführt werden (analog zu geschnürten Korsagen bzw. Schnürsenkeln), am Kleidungsstück 2 befestigt werden.

Das oben beschriebene, variabel auf unterschiedliche Körpermaße bzw. Konfektionsgrößen einstellbaren heizbare Kleidungsstück 2 kann eine beliebige Unter- und Oberbekleidung sein. Weiterhin kann das Kleidungsstück 2 für medizinische oder sportliche Anwendungen auf eine spezielle Körperpartie zugeschnitten sein (beispielsweise als Nierenwärmer, Arm- und Beinwärmer etc.).

### Bezugszeichenliste

- 1: Person
- 2: Elektrisch beheizbares Kleidungsstück
- 2': Weste
- 3: Körper
- 4: Innenschicht
- 6: Außenschicht
- 7, 7', 7": Heizelement
- 8: Heizgewebe
- 9: Polyesterfolie
- 10: Heizdraht
- 10': Metallfaden
- 11: Fixierelement
- 11': Reißverschluss
- 12, 12': Seitenteile des Reißverschlusses
- 13: Armausschnitt
- 14: Unterachselbereich der Weste
- 15: Rückenteil
- 16: Brustteil
- 17, 17': elektrische Verbindung Unterachselteil zu Brustteil/Rückenteil
- 18, 18': metallischer Krampen des Reißverschlusses
- 19, 19', 19": Kontaktierelement
- 20: Kabel
- 22, 22': Reißverschluss-Seitenteile
- 24: Unterachselstück
- 25, 25': Ränder des Unterachselstücks
- 27: Zusatz-Heizelement
- 30: Befestigungsmittel zur Fixierung von Zusatz-Heizelementen
- 30': Druckknopf
- 37: Zusatz-Heizelement
- 39: Kontaktierelement

## Patentansprüche

1. Elektrisch heizbares Kleidungsstück (2, 2') mit Heizelementen, die ein elektrisch leitfähiges Heizgewebe (8) umfassen (7, 7', 7", 27, 37), wobei das Kleidungsstück (2, 2') Fixierelemente (11) zur Variation der Form und/oder der Größe des Kleidungsstücks (2, 2') aufweist, **dadurch gekennzeichnet, dass** die Heizelemente (7, 7', 7", 27, 37) Kontaktierelemente (19, 19', 19") zum elektrischen Verbinden mit benachbarten Heizelementen (7, 7', 7", 27, 37) aufweisen, und dass die Kontaktierelemente (19) des Heizelements (7) mit den diesem Heizelement (7) zugeordneten Fixierelementen (11, 18) kurzgeschlossen sind.

2. Elektrisch heizbares Kleidungsstück (2, 2') nach Anspruch 1, **dadurch gekennzeichnet, dass** die Heizelemente (7, 7', 7") unlösbar mit dem Kleidungsstück (2) verbunden sind.

3. Elektrisch heizbares Kleidungsstück (2', 2') nach Anspruch 1 oder 2, **dadurch gekennzeichnet, dass** die Fixiermittel (11) als Befestigungsmittel (30) zur lösbaren Befestigung der Heizelemente (27, 37) ausgestaltet sind.

4. Elektrisch heizbares Kleidungsstück (2, 2') nach Anspruch 3, **dadurch gekennzeichnet, dass** die Befestigungsmittel (30) Reißverschlüsse und/oder Druckknöpfe (30') und/oder Klettmaterialien sind.

5. Elektrisch heizbares Kleidungsstück (2, 2') nach einem der Ansprüche 1 bis 4, **dadurch gekennzeichnet, dass** die Fixierelemente (11) durch mindestens einen Reißverschluss (11') gebildet sind, dessen Seitenteile (12, 12') beabstandet zueinander auf der Außenseite (6) oder der Innenseite (4) des Kleidungsstücks (2, 2') angeordnet sind.

6. Elektrisch heizbares Kleidungsstück (2, 2') nach einem der Ansprüche 1 bis 4, **dadurch gekennzeichnet, dass** die Fixierelemente (11) durch mindestens einen Reißverschluss (11') gebildet ist, zwischen dessen Seitenteilen (12, 12') ein mit Reißverschluss-Seitenteilen (22, 22') versehenes Heizelement (27) einfügbar Ist.

7. Elektrisch heizbares Kleidungsstück (2, 2') nach einem der Ansprüche 1 bis 3, **dadurch gekennzeichnet, dass** die Fixierelemente (11) durch Druckknöpfe gebildet sind.

8. Elektrisch heizbares Kleidungsstück (2, 2') nach einem der Ansprüche 1 bis 3, **dadurch gekennzeichnet, dass** die Fixierelemente (11) durch Klettverschlüsse gebildet sind.

9. Elektrisch heizbares Kleidungsstück (2, 2') nach einem der Ansprüche 1 bis 3, **dadurch gekennzeichnet, dass** die Fixierelemente (11) durch Haken und Ösen gebildet sind.

10. Elektrisch heizbares Kleidungsstück (2, 2') nach einem der Ansprüche 1 bis 3, **dadurch gekennzeichnet, dass** die Fixierelemente (11) durch In Ösen führbare Schnüre gebildet sind.

11. Elektrisch heizbares Kleidungsstück (2, 2') nach einem der vorangehenden Ansprüche, **dadurch gekennzeichnet, dass** die Heizelemente (7, 7', 7", 27, 37) sich in Bezug auf ihre spezifische Heizleistung unterscheiden.

12. Elektrisch heizbares Kleidungsstück (2, 2') nach einem der vorangehenden Ansprüche, **dadurch gekennzeichnet, dass** die Heizelemente (7, 7', 7", 27, 37) mit einem elastisch dehnbaren Trägergewebe (5) verbunden sind.

## Claims

1. Electrically heatable item of clothing (2, 2') with heating elements (7, 7', 7", 27, 37) comprising an electrically conductive heating fabric (8), wherein the item of clothing (2, 2') has fixing elements (11) for variation of the shape and or the size of the item of clothing (2, 2'), **characterised in that** the heating elements (7, 7', 7", 27, 37) have contacting elements (19, 19', 19") for electrical connection with neighbouring heating elements (7, 7', 7", 27, 37), and that the contacting elements (19) of the heating element (7) are short circuited with the fixing elements (11, 18) assigned to this heating element (7).

2. Electrically heatable item of clothing (2, 2') according to claim 1, **characterised in that** the heating elements (7, 7', 7") are connected non-releasably to the item of clothing (2).

3. Electrically heatable item of clothing (2', 2') according to claim 1 or 2, **characterised in that** the fixing means (11) is embodied as a fastening means (30) for releasable fastening of the heating elements (27, 37).

4. Electrically heatable item of clothing (2, 2') according to claim 3, **characterised in that** the fastening means (30) are zip fastenings and/or stud fixings (30') and/or hook and loop materials

5. Electrically heatable item of clothing (2, 2') according to one of claims 1 to 4, **characterised in that** the fixing elements (11) are formed by at least one zip fastener (11'), the side parts of which (12, 12') are disposed spaced apart from one another on the outer side (6) or the inner side (4) of the item of clothing (2, 2').

6. Electrically heatable item of clothing (2, 2') according to one of claims 1 to 4, **characterised in that** the fixing element (11) is formed by at least one zip fastening (11'), between the side parts (12, 12') of which a heating element (27) provided with zip fastening side parts (22, 22') is able to be inserted.

7. Electrically heatable item of clothing (2, 2') according to one of claims 1 to 3, **characterised in that** the fixing elements (11) are formed by stud fixings.

8. Electrically heatable item of clothing (2, 2') according to one of claims 1 to 3, **characterised in that** the fixing elements (11) are formed by hook and loop fasteners.

9. Electrically heatable item of clothing (2, 2') according to one of claims 1 to 3, **characterised in that** the fixing elements (11) are formed by hooks and eyes.

10. Electrically heatable item of clothing (2, 2') according to one of claims 1 to 3, **characterised in that** the fixing elements (11) are formed by laces able to be threaded in eyes.

11. Electrically heatable item of clothing (2, 2') according to one of the preceding claims, **characterised in that** the heating elements (7, 7', 7", 27, 37) differ in relation to their specific heating power.

12. Electrically heatable item of clothing (2, 2') according to one of the preceding claims, **characterised in that** the heating elements (7, 7', 7", 27, 37) are connected to an elastically expandable carrier fabric (5).

## Revendications

1. Vêtement (2, 2') pouvant être chauffé électriquement avec des éléments chauffants qui comprennent (7, 7', 7", 27, 37) un tissu chauffant (8) électroconducteur, où le vêtement (2, 2') présente des éléments de fixation (11) s'adaptant à la variation de la forme et/ou de la taille du vêtement (2, 2'), **caractérisé en ce que** les éléments chauffants (7, 7', 7", 27, 37) présentent des éléments de contact (19, 19', 19") pour la connexion électrique avec des éléments chauffants adjacents (7, 7', 7", 27, 37), et **en ce que** les éléments de contact (19) de l'élément chauffant (7) sont court-circuités par les éléments fixateurs (11, 18) associés à cet élément chauffant (7).

2. Vêtement (2, 2') pouvant être chauffé électriquement selon la revendication 1, **caractérisé en ce que** les éléments chauffants (7, 7', 7") sont reliés au vêtement (2) de façon indétachable.

3. Vêtement (2, 2') pouvant être chauffé électriquement selon la revendication 1 ou 2, **caractérisé en ce que** les moyens fixateurs (11) sont conçus comme des moyens de fixation (30) servant à la fixation détachable des éléments chauffants (27, 37).

4. Vêtement (2, 2') pouvant être chauffé électriquement selon la revendication 3, **caractérisé en ce que** les moyens de fixation (30) sont des fermetures Eclair et/ou des boutons-pression (30') et/ou des matières auto-agrippantes.

5. Vêtement (2, 2') pouvant être chauffé électriquement selon l'une quelconque des revendications 1 à 4, **caractérisé en ce que** les éléments fixateurs (11) sont formés par au moins une fermeture Eclair (11') dont les parties latérales (12, 12') espacées l'une de l'autre sont disposées sur le côté extérieur (6) ou sur le côté intérieur (4) du vêtement (2, 2').

6. Vêtement (2, 2') pouvant être chauffé électriquement selon l'une quelconque des revendications 1 à 4, **caractérisé en ce que** les éléments fixateurs (11) sont formés par au moins une fermeture Eclair (11') entre les parties latérales (12, 12') de laquelle peut être inséré un élément chauffant (27) doté de parties latérales (22, 22') de la fermeture Eclair.

7. Vêtement (2, 2') pouvant être chauffé électriquement selon l'une quelconque des revendications 1 à 3, **caractérisé en ce que** les éléments fixateurs (11) sont formés par des boutons-pression.

8. Vêtement (2, 2') pouvant être chauffé électriquement selon l'une quelconque des revendications 1 à 3, **caractérisé en ce que** les éléments fixateurs (11) sont formés par des fermetures auto-agrippantes.

9. Vêtement (2; 2') pouvant être chauffé électriquement selon l'une quelconque des revendications 1 à 3, **caractérisé en ce que** les éléments fixateurs (11) sont formés par des crochets et des oeillets.

10. Vêtement (2, 2') pouvant être chauffé électriquement selon l'une quelconque des revendications 1 à 3, **caractérisé en ce que** les éléments fixateurs (11) sont formés par des cordons pouvant passer dans des oeillets.

11. Vêtement (2, 2') pouvant être chauffé électriquement selon l'une quelconque des revendications précédentes, **caractérisé en ce que** les éléments chauffants (7, 7', 7", 27, 37) se différencient par rapport à leur puissance de chauffage spécifique.

12. Vêtement (2, 2') pouvant être chauffé électriquement selon l'une quelconque des revendications précédentes, **caractérisé en ce que** les éléments chauffants (7, 7', 7", 27, 37) sont reliés à un tissu support (5) élastiquement extensible.
